# EUROPÄISCHE PATENTSCHRIFT

(11) **EP 1 397 138 B1**
(45) Veröffentlichungstag und Bekanntmachung des Hinweises auf die Patenterteilung: **05.01.2005**
(21) Anmeldenummer: 02754688.6
(22) Anmeldetag: 15.06.2002
(51) Int. Cl.: A61K 31/505, A61K 31/485, A61K 31/185, A61K 31/519, A61P 25/30, A61P 25/32

(54) **WIRKSTOFF-KOMBINATION (z.B. Galanthamin oder Desoxypeganin mit Acamprosat oder Memantin) ZUR MEDIKAMENTÖSEN SUCHT- ODER RAUSCHMITTELTHERAPIE**
ACTIVE INGREDIENT COMBINATION (e.g. galanthamine or desoxypeganine with acamprosate or Memantine) FOR TREATING A DEPENDENCE ON ADDICTIVE SUBSTANCES OR NARCOTICS
ASSOCIATION DE PRINCIPES ACTIFS (par ex. de la galanthamine ou la désoxypéganine avec de l'acamprosate ou la mémantine) DESTINEE AU TRAITEMENT D'UNE INTOXICATION A DES SUBSTANCES ENTRAINANT UNE DEPENDANCE OU A DES STUPEFIANTS

(30) Priorität: 18.06.2001 DE 10129265
(43) Veröffentlichungstag der Anmeldung: 17.03.2004
(73) Patentinhaber: HF ARZNEIMITTELFORSCHUNG GmbH, 59368 Werne (DE)
(72) Erfinder: MOORMANN, Joachim, 59368 Werne (DE); MUCKE, Hermann, A-1160 Wien (AT); OPITZ, Klaus, 48157 Münster (DE)
(74) Vertreter: Flaccus, Rolf-Dieter, Dr.
(86) Internationale Anmeldenummer: PCT/EP2002/006630
(87) Internationale Veröffentlichungsnummer: WO 2002/102388

(56) Entgegenhaltungen:
- EP-A- 0 424 179
- EP-A- 0 449 247
- EP-A- 0 945 133
- WO-A-99/54280
- DE-A- 19 906 974
- STROMBERG M F ET AL: "Effect of acamprosate and naltrexone, alone or in combination, on ethanol consumption." ALCOHOL (FAYETTEVILLE, N.Y.) UNITED STATES FEB 2001, Bd. 23, Nr. 2, Februar 2001 (2001-02), Seiten 109-116, XP001120787 ISSN: 0741-8329 in der Anmeldung erwähnt

## Beschreibung

Die vorliegende Erfindung betrifft Wirkstoff-Kombinationen und deren Verwendung zur medikamentösen Sucht- oder Rauschmitteltherapie, insbesondere Alkohol betreffend. Dabei besteht die Wirkstoff-Kombination aus zumindest einem Modulator des cholinergen Systems mit zumindest einer antiexzitatorisch wirkenden Substanz. Des weiteren betrifft die vorliegende Erfindung die Verwendung der angesprochenen Wirkstoff-Kombinationen zur Herstellung von Arzneimitteln, die zur Therapie des Konsums von Sucht- oder Rauschmitteln, insbesondere des Konsums von Alkohol, beitragen.

Die Zuführung von Sucht- und Rauschmitteln, insbesondere von Alkohol, führt bekanntermaßen zu Symptomen wie Wahrnehmungsstörungen, Gedächtnisverlust, Beeinträchtigung kognitiver Fähigkeiten, allgemeiner Kontrollverlust, Aggressivität und Beeinträchtigung der Bewegungskoordination. Wird das Rauschmittel bewußt zugeführt, so sind derartige Wirkungen zwar von der rauschmittelkonsumierenden Person beabsichtigt, jedoch werden sie unter bestimmten Voraussetzungen auch als nachteilig empfunden. Hinzu kommt, daß der Schweregrad und die zeitliche Fortdauer dieser Symptome variieren kann und für den Rauschmittelkonsumenten im voraus oft nur schwer einzuschätzen ist.

Insbesondere bei chronischer Abhängigkeit und fortgesetztem Rauschmittelmißbrauch kommt es nicht nur zu den allgemein bekannten Organschädigungen, sondern es treten auch anhaltende Ausfallserscheinungen auf, welche z. B. die kognitiven Leistungen, insbesondere Gedächtnisleistungen, beeinträchtigen. Dies kann auch zu sporadischen oder anhaltenden Demenz-Zuständen führen. Auch die bereits genannten psychiatrischen Symptome, wie z. B. ein allgemeiner Kontrollverlust, können sich chronisch manifestieren. Diese chronischen Folgeerscheinungen des Alkoholmißbrauchs - die bei anderen Rauschmittelabhängigkeiten in ähnlicher Weise auftreten - stellen ein erhebliches Hindernis für eine erfolgreiche Durchführung von Entwöhnungstherapien dar. So ist bekannt, daß der durch chronischen Alkoholmißbrauch bedingte Kontrollverlust eine Abstinenz-Unfähigkeit des betroffenen Alkoholkranken bewirkt. Dies ist die Hauptursache dafür, daß selbst entwöhnte Alkoholiker zu Rückfällen neigen, mit meist schwerwiegenden Folgen. Aus dieser Beobachtung wurde der Grundsatz abgeleitet, daß ein "kontrolliertes Trinken" bei Abhängigen nicht möglich ist.

Es ist ferner bekannt, daß es hinsichtlich des Rauschmittelkonsumverhaltens große individuelle Unterschiede gibt, aufgrund derer man beispielsweise Alkoholiker in unterschiedliche Trinker-Kategorien einteilt.
Bei bestimmten Alkohol-Konsumenten besteht das Problem, daß nach Überschreiten einer bestimmten individuellen Schwellendosis rasch ein allgemeiner Kontrollverlust mit den oben erwähnten negativen Begleiterscheinungen eintritt. Die betroffenen Personen sind meist nicht in der Lage, das Erreichen ihrer individuellen Schwellendosis oder überhaupt ihr persönliches Rückfall-Risiko rechtzeitig zu erkennen. Aufgrund des dadurch bewirkten Kontrollverlustes, kommt es häufig zu weiterem, exzessiven Alkoholgenuß. Besonders betroffen sind Personen, die bereits Entzugstherapien hinter sich haben und auf diese Weise rückfällig werden.

Bekanntermaßen hat der durch chronischen Suchtmittelmißbrauch verursachte Kontrollverlust, wie auch die Beeinträchtigung der Gedächtnisleistungen (bis hin zur Demenz) für den Betroffenen wie auch für seine Umgebung oft weiterreichende Folgen, wie z. B. Unfähigkeit zur Berufsausübung, Unfähigkeit zur Strukturierung des Tagesablaufs, Unfähigkeit zur Aufnahme und Pflege sozialer Kontakte und daraus folgend soziale Ausgrenzung.

Die suchtmittelbedingten Ausfallserscheinungen, z. B. die Beeinträchtigung der kognitiven Leistungen, dauern selbst nach einer erfolgreich abgeschlossenen Entzugstherapie oft noch an. Weitere bei Alkoholmißbrauch oder bei Mißbrauch anderer Suchtmittel auftretende psychiatrische oder zerebrale Störungen sind z. B.: Wahrnehmungs- oder Sinnestäuschungen, Amnesie, Bewußtseinsveränderungen, formale Denkstörungen, Merkfähigkeitsstörungen, Wahnvorstellungen, Konfabulationen, Desorientiertheit, Erregungszustände.

Zur medikamentösen Therapie des Alkoholmißbrauchs sind derzeit in europäischen Staaten und/oder in den Vereinigten Staaten von Amerika fünf Präparate zugelassen. Am längsten findet Bis-(diethylthiocarbamoyl)disulfid (Disulfiram, Antabus®) Verwendung, das durch Blockierung der Aldehyddehydrogenase zu einer Anhäufung toxischer Endprodukte des Alkoholabbaus und in dessen Folge zu Übelkeit nach Alkoholkonsum führt. Trotz der aversiven Wirkung wird das eigentliche Alkoholverlangen nicht beeinflußt. Tiapride, ein Antagonist des Dopamins, der an den Dopaminrezeptor-Subtypen D2 und D3 wirkt, hat kaum eine praktische Bedeutung erlangt. In weit größerem Umfang werden der Opiatrezeptor-Antagonist Naltrexon (ReVia®, DuPont, Trexan®) und das auf komplexe Weise wirkende Acamprosat (Campral®, Merck AG; Aortal®) verwendet, um Rückfälle in den Alkoholmißbrauch nach erfolgtem Alkoholentzug zu verhindern. Seit kurzem steht in einigen europäischen Ländern auch Gamma-Hydroxybutyrat (z.B. Alcover®, Gerot Pharmazeutika) zur Verfügung. Naltrexon und Gamma-Hydroxybutyrat rufen jedoch erhebliche gastrointestinale und psychomotorische Nebenwirkungen hervor, die die Compliance mit der Therapie beeinträchtigen. Naltrexon ist zudem durch seine geringe orale Bioverfügbarkeit gekennzeichnet und überdies hepatotoxisch, während Gamma-Hydroxybutyrat selbst über Suchtpotential verfügt.

Dennoch sind die langfristigen Erfolge sämtlicher zugelassener Pharmaka insgesamt als sehr beschränkt zu bezeichnen, da sie bei der Mehrzahl der Patienten nur marginale Verzögerungen des Rückfalls nach Entzug bzw. nur eine klinisch unbedeutende Reduktion der Alkoholmenge bewirken. Die Tatsache, daß durchschnittlich nur ca. 30% aller Patienten ein Jahr nach der Entzugsbehandlung noch abstinent sind, ist durch diese Medikamente nicht nachhaltig beeinflußt worden. Eine Therapie der frühen Stadien der oft Jahrzehnte überspannenden Entwicklung des Alkoholismus erfordert zudem besonders nebenwirkungsarme Medikamente, da bei den sogenannten "Social Drinkers" angesichts des noch geringen Leidensdruckes kaum Einsicht in die Problematik ihres Trinkverhaltens und daher wenig Bereitschaft zum Ertragen von Nebenwirkungen einer medikamentösen Alkoholtherapie besteht. Es fehlt daher seit Jahren nicht an Versuchen, pharmakologische Verbesserungen in die Alkoholtherapie einzuführen, wobei die vorgeschlagenen Substanzen und Substanzkombinationen sowie ihre Verwendung in der Alkoholismustherapie, mit wenigen Ausnahmen, schon vorher bekannt waren. Als Beispiel für eine derartige Kombination von Wirkstoffen, von denen jeder seit Jahren in der Therapie des Alkoholabusus Verwendung findet, ist die in EP 0 945 133 beschriebene gemeinsame Verwendung von Acamprosat und Naltrexon. Nach neuesten Studien (*Neurosci*. *Behav*. 2001; 23(2): 109-118) ist dieser Kombination jedoch keine synergistische Wirkung zuzuschreiben.

Als Alternative zur Behandlung des Alkoholabusus wird in den Druckschriften DE 40 10 079 und US 5 519 017 die Verwendung von Galanthamin vorgeschlagen, das das Verlangen nach Nikotin bzw. Alkohol unterdrücken soll. Darüberhinaus beschreibt US 5 932 238 ein für Galanthamin geeignetes transdermales therapeutisches System.

Galanthamin wird auch zur Behandlung der Poliomyelitis, der Alzheimerschen Krankheit und verschiedener Erkrankungen des Nervensystems eingesetzt, sowie zur Behandlung des Engwinkelglaukoms.

Galanthamin bzw. Galantamin (4a,5,9,10,11,12-Hexahydro-3-methoxy-11-methyl-6-H-benzofuro-(3a,3,2-ef)-(2)-benzazepin-6-ol) ist ein tetracyclisches Alkaloid, das bei bestimmten Pflanzen, insbesondere bei Amaryllidaceen, vorkommt. Es kann aus diesen Pflanzen mittels bekannter Verfahren gewonnen werden (z. B. gemäß DE 195 09 663 A1 oder DE-PS 11 93 061) oder auf synthetischem Wege (z. B. Kametani et al., *Chem. Soc. C*. 6, 1043-1047 (1971) oder Shimizu et al., *Heterocycles* 8, 277-282 (1977)) hergestellt werden.

Aufgrund seiner pharmakologischen Eigenschaften wird Galanthamin zur Gruppe der reversibel wirkenden Cholinesterasehemmstoffe gezählt. Gleichzeitig stimuliert Galanthamin auch die Ausschüttung des Neurotransmitters Acetylcholin durch direkte Stimulation der präsynaptischen nikotinischen Acetylcholinrezeptoren. Ein analoger Vorgang erfolgt auch auf dopaminergen präsynaptischen Nervenendigungen, wo es die Ausschüttung von Dopamin fördert. Diese Eigenschaften des Galanthamin sollen nach gängigen Theorien das "Craving" nach Alkohol unabhängig von kognitiver Kontrolle reduzieren, was den theoretischen Unterbau der Druckschriften DE-40 10 079 und US 5 932 238 bildet.

Der für Galanthamin beschriebene kombinierte direkte cholinerge und indirekte dopaminerge Effekt läßt sich auch mit Substanzen erzielen, die gleichzeitig die Acetylcholinesterase und die Monoaminoxidase inhibieren. Dies ist beispielsweise beim Desoxypeganin der Fall, das insbesondere in der älteren Literatur auch als Desoxyvasicin bezeichnet wird. Daher wird gemäß DE 199 06 974 auch Desoxypeganin für die Therapie des Alkoholmißbrauchs beansprucht. Darüberhinaus wurde vorgeschlagen, Desoxypeganin ebenfalls zur medikamentösen Therapie der Alzheimer'schen Demenz, zur Behandlung der Nikotinabhängigkeit durch Verminderung des Verlangens nach Nikotin oder zur Substitutionstherapie von Drogensüchtigen bzw. zur Behandlung von Entzugserscheinungen während einer Entzugstherapie anzuwenden. Außerdem kann Desoxypeganin als Cholinesterasehemmer als Antidot oder Prophylaktikum bei Vergiftungen durch organische Phosphorsäureester eingesetzt werden, wobei es die zerebrale Wirkung cholinerger Gifte antagonisiert.

Desoxypeganin (1,2,3,9-Tetrahydropyrrolo[2,1-b]chinazolin) ist ein Alkaloid der Summenformel C₁₁H₁₂N₂, das in Pflanzen aus der Familie der Zygophyllaceae enthalten ist. Die Gewinnung von Desoxypeganin erfolgt vorzugsweise durch Isolierung aus der Steppenraute (*Peganum harmala*) oder durch Synthese.

Trotz ihrer doppelten Wirkmechanismen sind Galanthamin und Desoxypeganin nur beschränkt geeignet, das Verlangen nach Sucht- oder Rauschmitteln wirksam zu unterdrücken. Der Grund dafür dürfte darin liegen, daß das Verlangen nach Alkohol nach derzeitigem Wissensstand von einer neuronalen Übererregung wesentlich mitverursacht wird. Diese Übererregung treibt die Suchtabhängigen zu immer neuem Trinken, da durch die akute Intoxikation mit Alkohol diese Übererregung des Nervensystems dämpft wird. Weder Galanthamin noch Desoxypeganin beeinflussen die chronische neuronale Übererregung, so daß eine Unterdrückung des "Cravings" allein durch diese Substanzen nicht möglich ist.

Ziel der vorliegenden Erfindung war daher die Bereitstellung von Arzneimitteln, durch die die alkoholinduzierte Übererregung gedämpft wird, ohne jedoch die physiologische exzitatorische Reizleitung in relevantem Ausmaß zu beeinträchtigen, damit die so erhaltenen Arzneimittel keine nicht vertretbaren Nebenwirkungen, wie z. B. starke Sedierung oder Beeinträchtigung der Kognition, aufweisen, um den Alkoholkonsum zu verringern.

Überraschenderweise wurde gefunden, daß die der vorliegenden Erfindung zugrundeliegende Aufgabe durch die Kombination eines Modulators des cholinergen Systems mit antiexzitatorisch wirkenden Substanzen bestimmter Untergruppen besonders gut gelöst werden kann.

Erfindungsgemäß können Modulatoren des cholinergen Systems verwendet werden, die neben ihrer inhibitorischen Wirkung auf Cholinesterasen auch auf dopaminerge Nervenendigungen wirken. Dies kann beispielsweise durch Substanzen erfolgen, die als Cholinesteraseinhibitoren auch nikotinische Acetylcholinrezeptoren an den präsynaptischen Nervenendigungen cholinerger und domapinerger Nervenendigungen direkt stimulieren oder durch Substanzen, die gleichzeitig die Acetylcholinesterase und die Monoaminoxidase inhibieren.

Bevorzugt werden als Modulatoren des cholinergen Systems mit den zuvor angesprochenen Eigenschaften Galanthamin oder Desoxypeganin oder deren pharmakologisch akzeptable Derivate verwendet. Es versteht sich für den Fachmann von selbst, daß Galanthamin oder Desoxypeganin in Form ihrer freien Basen oder in Form ihrer bekannten Salze oder Derivate verwendet werden. So können beispielsweise anstelle der Salze bzw. Additionsverbindungen des Galanthamins auch sämtliche in der wissenschaftlichen Literatur und in Patentschriften aufgeführten bzw. beanspruchten Derivate des Galanthamins verwendet werden, insoweit diese entweder Inhibitoren der Cholinesterase-Enzyme oder Modulatoren der nikotinischen Acetylcholinrezeptoren sind bzw. beide pharmakologischen Aktivitäten kombiniert ausüben. Darunter sind insbesondere zu verstehen:
- Die in den Patentschriften der Familien WO-9612692 / EP-0787115 / US-6043359 sowie WO-9740049 / EP-0897387 und WO-032199 (Waldheim Pharmazeutika GmbH. bzw. Sanochemia Pharmazeutika AG) angeführten Verbindungen, darunter insbesondere:
   (-)-N-Demethylgalanthamin;
   (-)-(N-Demethyl)-N-allylgalanthamin;
   (-)-(6-Demethoxy)-6-hydroxygalanthamin (SPH-1088);
   (+/-) N-Demethylgalanthamin N-*tert*-butyl carboxamid (SPH-1221);
   (-) N-Demethylgalanthamin N-*tert*-butyl carboxamid
- Die in den Patentschriften der Familien EP-0648771 und EP-0653427 (Hoechst Roussel Pharmaceuticals Inc.) sowie *Drugs Fut*. 21(6), 621-635 (1996) und *J*. *Pharmacol*. *Exp*. *Ther*. 277(2), 728-738 (1996) angeführten Verbindungen, darunter insbesondere:
   (-)-6-O-Demethylgalanthamin;
   (-)-(6-O-Acetyl)-6-O-demethylgalanthamin (P11012);
   (-)-(6-O-Demethyl)-6-O-[(adamantan-1-yl)carbonyl]galanthamin (P11149);
   (-)-(6-O-Demethyl)-6-O-(triethylsilyl)galanthamin;
   (-)-(6-O-Demethyl)-6-O-(triisopropylsilyl)galanthamin;
   (-)-(6-O-Demethyl)-6-O-(trimethylsilyl)galanthamin;
- Die in den Patentschriften der Familien WO-9703987 / EP-0839149 / US-5958903 (Societe de Conseils de Recherches et D'Applications Scientifiques, S.C.R.A.S) angeführten Verbindungen, darunter insbesondere:
   (6-O-Demethyl)-6-O-(8'-phtalimidooctyl)galanthaminium bromohydrat;
   (6-O-Demethyl)-6-O-(4'-phtalimidobutyl)galanthaminium bromohydrat;
   (6-O-Demethyl)-6-O-(10'-phtalimidodecyl)galanthaminium bromohydrat;
   (6-O-Demethyl)-6-O-(12'-phtalimidododecyl)galanthaminium bromohydrat;
   10-N-Demethyl-10-N-(10'-phtalimidobutyl)galanthaminium trifluoroacetat;
   10-N-Demethyl-10-N-(10'-phtalimidohexyl)galanthaminium trifluoroacetat;
   10-N-Demethyl-10-N-(10'-phtalimidooctyl)galanthaminium bromohydrat;
   10-N-Demethyl-10-N-(10'-phtalimidododecyl)galanthaminium bromohydrat;
   10-N-Demethyl-10-N-(12'-phtalimidododecyl)galanthaminium bromohydrat;
   10-N-Demethyl-10-N-(6'-pyrollohexyl)galanthaminium bromohydrat
- Die unter anderem in der Veröffentlichung *Bioorg. Med*. *Chem*. 6(10), 1835-1850 (1998) beschriebenen (-)N,N'-Demethyl-N,N'-Bis-Galantaminderivate folgender Strukturformel, wobei die Überbrückungsgruppe ("Alkyl-Spacer") zwischen den Stickstoffatomen der beiden Galanthamin-Moleküle 3-10 CH₂-Gruppen lang sein kann und unabhängig davon eines der beiden Galanthamin-Moleküle eine positive Ladung am Stickstoffatom tragen kann (Galanthaminium-Kation): 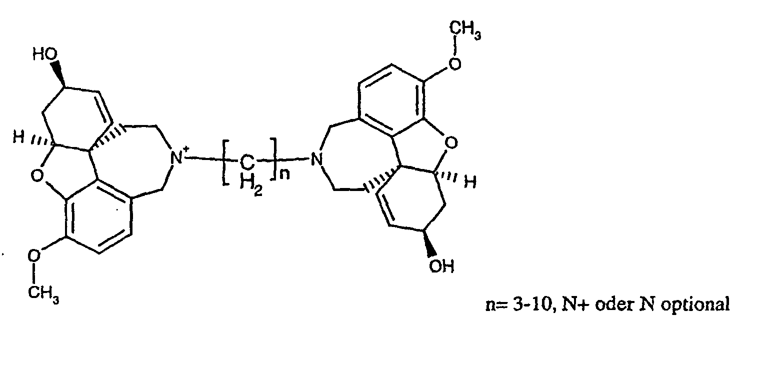
- Das unter anderem in der Veröffentlichung *J*. *Cerebral Blood Flow Metab*. 19(Suppl. 1), S19 (1999) sowie in *Proteins* 42, 182-191 (2001) beschriebene (-)N-Demethyl-N-(3-piperidinopropyl)galanthamin (SPH-1286) sowie dessen Analoga mit "Alkyl-Spacern" von bis zu 10 CH₂-Gruppen Länge:

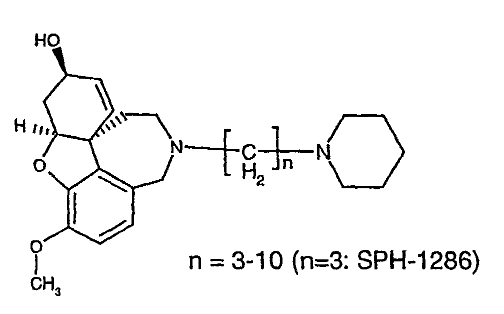

In gleicher Weise sind anstelle von Desoxypeganin auch dessen in der Literatur beschriebene Derivate zu verstehen, insoweit diese gleichzeitig Hemmstoffe der Acetylcholinesterase und von Monoaminooxidasen sind. Dazu zählen das in *Syntbetic Communs*. 25(4), 569-572 (1995) beschriebene 7-Bromodesoxypeganin, ebenso die in *Drug Des*. *Disc*. 14, 1-14 (1996) beschriebenen 7-Halo-6-hydroxy-5-methoxydesoxypeganine der allgemeinen Formel 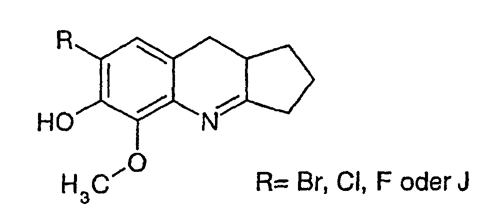
7-Bromo-6-hydroxy-5-methoxydesoxypeganin
7-Chloro-6-hydroxy-5-methoxydesoxypeganin
7-Fluoro-6-hydroxy-5-methoxydesoxypeganin
7-Jodo-6-hydroxy-5-methoxydesoxypeganin

Des weiteren sind auch die in *Ind*. *J*. *Chem*. 24B, 789-790 (1985) beschriebenen Derivative des Desoxypeganins verwendbar.

Die verabreichte Einzeldosis von Galanthamin oder einem seiner pharmakologisch akzeptablen Salze oder Derivate liegt vorzugsweise im Bereich von 1 bis 50 mg, wohingegen die verabreichte Einzeldosis von Desoxypeganin oder einem seiner pharmakologisch akzeptablen Salze oder Derivate vorzugsweise im Bereich von 10 bis 500 mg liegt.

Nach der Erfindung werden Galanthamin oder Desoxypeganin oder eines ihrer pharmakologisch akzeptablen Salze oder Derivate mit zumindest einer antiexzitatorsich wirkenden Substanz kombiniert.
Besonders vorteilhaft wird die Aufgabe durch eine Kombination mit Vertretern bestimmter Untergruppen antiexzitatorsich wirkender und pharmakologisch akzeptabler Verbindungen erfüllt. Dazu zählen vor allem
- die zustandsselektiven, nicht-kompetitiven Antagonisten des aktivierten NMDA-Rezeptors, darunter besonders die in der Klasse der Adamantanderivate zu findenden Substanzen (wie z.B. Memantine) und bestimmte Aminoalkylcyclohexanderivate, sowie
- Verbindungen, die neben einem Antagonismus an NMDA-Rezeptoren auch eine verstärkende Wirkung auf das zentrale GABAerge System und damit eine weitere dämpfende Wirkung auf das Zentralnervensystem ausüben, worunter Verbindungen aus der strukturellen Klasse der linearen aliphatischen Sulfon- und Aminosulfonsäuren wie z.B. Derivate des Taurins, insbesondere Acamprosat, zu verstehen sind; und
- Verbindungen, die metabotrope Glutamatrezeptoren derart modulieren, daß die neuronale Übererregung in der oben beschriebenen Weise gedämpft wird.

Es ist offensichtlich, dass anstelle von Acamprosat Salze der mit diesem strukturell verwandten Aminoalkansulfonsäurederivate mit vergleichbarer pharmakologischer Wirksamkeit eingesetzt werden können, vor allem sämtliche in WO-9937606 (Lipha S.A.) beanspruchten, worunter sich insbesondere das Magnesiumsalz der 3-(2-Methylpropanoylamino)propansulfonsäure befindet. Gleiches gilt für Derivate von Memantine, worunter sämtliche Adamantanderivate zu verstehen sind, die an die aktivierte Form des N-Methyl-D-aspartat-Rezeptors binden und die Effekte exzitatorisch wirkender Liganden auf diesen blockieren. Insbesondere sind dies sonstige 1-Aminoadamantanderivate wie Amantadine, jedoch auch Memantine-Analoga mit den gleichen pharmakologischen Eigenschaften, wie etwa 1-Amino-1,3,3,5,5-pentamethyl-cyclohexan (MRZ 2/579).

Weiterhin werden in den pharmazeutischen Zubereitungen Galanthamin oder Desoxypeganin oder ihre pharmakologisch akzeptablen Salze oder Derivate gemeinsam mit Antagonisten verschiedener Klassen von zentralen metabotropen Glutamatrezeptoren (mGluR) kombiniert. Als mGluR-Antagonisten eignen sich insbesondere die in WO-0026198 und WO-0026199 beanspruchten Verbindungen 3,6-Dihydro-3,5-dimethyl-6-(4-ethoxyphenyl)-2-(4-methanesulfonylaminophenylsulfonyl)-2H-1,2-oxazin bzw. 2-(4-Acetylaminobenzensulfonyl)-3,6-dihydro-3,5-dimethyl-6-(4-methoxyphenyl)-2H-1,2-oxazin; sowie das in WO-0069816 beanspruchte 3-(3-Chlorobenzoylamino)-1-[2-(3-chlorophenyl)-ethyl]-3-methylpyrrolidin-2-thion und seine in diesem Dokument genannten Verwandten.

Die verabreichte Einzeldosis von Acamprosat oder einem seiner pharmakologisch akzeptablen Salze oder Derivate liegt vorzugsweise im Bereich von 100 bis 5000 mg, wohingegen die verabreichte Einzeldosis von Memantine oder einem seiner pharmakologisch akzeptablen Salze oder Derivate vorzugsweise im Bereich von 1 bis 50 mg liegt. Die Dosierung der Antagonisten verschiedener Klassen von zentralen metabotropen Glutamatrezeptoren kann zwischen 0,1 mg und 100 mg pro Arzneimitteleinheit liegen.

Die Arzneiformen, welche gemäß vorliegender Erfindung zur Verabreichung einer Kombination von Modulatoren des cholinergen Systems mit einer antiexzitatorisch wirkenden Substanz oder einem Modulator metabotroper Glutamatrezeptoren verwendet werden, können einen oder mehrere folgender Zusatzstoffe enthalten:
- Antioxydantien, Synergisten, Stabilisatoren;
- Konservierungsmittel;
- Geschmackskorrigentien;
- Färbemittel;
- Lösemittel, Lösungsvermittler;
- Tenside (Emulgatoren, Solubilisatoren, Benetzer, Entschäumer);
- Viskositäts- und Konsistenzbeeinflusser, Gelbildner;
- Resorptionsbeschleuniger;
- Adsorptionsmittel, Feuchthaltemittel, Gleitmittel;
- Zerfalls- und Lösungsbeeinflusser, Füllmittel (Streckmittel), Peptisatoren;
- Freisetzungsverzögerer.

Diese Aufzählung ist nicht abschließend; die in Frage kommenden physiologisch unbedenklichen Substanzen sind dem Fachmann bekannt.

Die Verabreichung einer Kombination von Modulatoren des cholinergen Systems mit einer antiexzitatorisch wirkenden Substanz kann oral oder parenteral erfolgen. Für die orale Verabreichung können Arzneimittel in bekannten Darreichungsformen, wie Tabletten, Dragees oder Pastillen, hergestellt werden. Daneben kommen auch flüssige oder halbflüssige Darreichungsformen in Betracht, wobei der Wirkstoff als Lösung oder Suspension vorliegt. Als Löse- oder Suspendierungsmittel können Wasser, wässrige Medien oder pharmakologisch unbedenkliche Öle (vegetabilische oder Mineralöle) verwendet werden.

Vorzugsweise sind die eine Kombination von Modulatoren des cholinergen Systems mit einer antiexzitatorisch wirkenden Substanz enthaltenden Arzneimittel als Depot-Arzneimittel formuliert, welche in der Lage sind, diesen Wirkstoff über einen längeren Zeitraum in kontrollierter Weise an den Organismus abzugeben.

Darüber hinaus kann die Verabreichung einer Kombination von Modulatoren des cholinergen Systems mit einer antiexzitatorisch wirkenden Substanz nach der Erfindung auch auf parenteralem Wege erfolgen. Hierzu können transdermale oder transmucosale Darreichungsformen besonders vorteilhaft für die erfindungsgemäße Verabreichung einer Kombination von Modulatoren des cholinergen Systems mit einer antiexzitatorisch wirkenden Substanz verwendet werden, insbesondere klebende transdermale therapeutische Systeme (Wirkstoffpflaster). Diese ermöglichen es, den Wirkstoff über einen längeren Zeitraum in kontrollierter Weise über die Haut an den zu behandelnden Patienten abzugeben.
Ein weiterer Vorteil ist, daß eine mißbräuchliche Verwendung bei parenteralen Applikationsformen weniger leicht möglich ist als bei oralen Darreichungsformen. Durch die vorgegebene Wirkstoff-Freisetzungsfläche und die vorbestimmte Freisetzungsrate kann eine Überdosierung seitens des Patienten weitgehend ausgeschlossen werden. Außerdem sind transdermale Darreichungsformen aufgrund weiterer Eigenschaften sehr vorteilhaft, z. B. Vermeidung des First-Pass-Effektes oder eine bessere, gleichmässigere Steuerung des Blutspiegels.

Derartige transdermale, eine Kombination von Modulatoren des cholinergen Systems mit einer antiexzitatorisch wirkenden Substanz enthaltenden Systeme weisen üblicherweise eine wirkstoffhaltige, haftklebende Polymermatrix auf, die auf der hautfernen Seite von einer wirkstoffundurchlässigen Rückschicht bedeckt ist, und deren klebende, wirkstoffabgebende Oberfläche vor der Applikation mit einer ablösbaren Schutzschicht bedeckt ist. Die Herstellung solcher Systeme und die dabei verwendbaren Grundstoffe und Hilfsstoffe sind dem Fachmann grundsätzlich bekannt; beispielsweise wird der Aufbau solcher transdermalen therapeutischen Systeme in den deutschen Patenten DE 33 15 272 und DE 38 43 239 oder in den US-Patenten 4 769 028, 5 089 267, 3 742 951, 3 797 494, 3 996 934 und 4 031 894 beschrieben.

Die erfindungsgemäße Kombination eines Modulators des cholinergen Systems mit einer antiexzitatorisch wirkenden Substanz kann bei der Therapie von Suchtmittel- und Rauschmittelmißbrauch verwendet werden, um den Konsum der Suchtmittel bzw. Rauschmittel zu verringern.

Die erfindungsgemäße Kombination eines Modulators des cholinergen Systems mit einer antiexzitatorisch wirkenden Substanz kann zur Herstellung von Arzneimitteln zur Therapie von Suchtmittel- und Rauschmittelmißbrauch verwendet werden, um den Konsum der Suchtmittel bzw. Rauschmittel zu verringern, insbesondere den Konsum von Alkohol.

In beispielhafter Weise wird die Aufgabe der Erfindung wie folgt gelöst, ohne daß der Umfang der Erfindung durch diese beispielhafte Aufzählung eingeschränkt werden soll.

### Beispiel 1

Oral oder transdermal zu verabreichendes Medikament, welches pro Einzeldosis 1 mg bis 50 mg Galanthamin in Form eines seiner pharmakologisch akzeptablen Salze, vorzugsweise in Form seines Hydrobromids, oder Additionsverbindungen und 100 mg bis 5000 mg eines pharmakologisch akzeptablen Salzes des N-Acetylhomotaurins, vorzugsweise des Kaliumsalzes, enthält.

### Beispiel 2

Oral oder transdermal zu verabreichendes Medikament, welches pro Einzeldosis 1 mg bis 50 mg Galanthamin in Form eines seiner pharmakologisch akzeptablen Salze, vorzugsweise in Form seines Hydrobromids, oder Additionsverbindungen und 1 mg bis 50 mg 1-Amino-3,5-dimethyl-adamantane enthält.

### Beispiel 3

Oral oder transdermal zu verabreichendes Medikament, welches pro Einzeldosis 10 mg bis 500 mg Desoxypeganin in Form eines seiner pharmakologisch akzeptablen Salze, vorzugsweise in Form seines Hydrochlorids, oder Additionsverbindungen und 100 mg bis 5000 mg eines pharmakologisch akzeptablen Salzes des N-Acetylhomotaurins, vorzugsweise des Kaliumsalzes, enthält.

### Beispiel 4

Oral oder transdermal zu verabreichendes Medikament, welches pro Einzeldosis 10 mg bis 500 mg Desoxypeganin in Form eines seiner pharmakologisch akzeptablen Salze, vorzugsweise in Form seines Hydrochlorids, oder Additionsverbindungen und 1 mg bis 50 mg 1-Amino-3,5-dimethyl-adamantan enthält.

## Patentansprüche

1. Wirkstoff-Kombination aus zumindest einem Modulator das cholinergen Systems mit zumindest einer antiexzitatorisch wirkenden Substanz zur medikamentösen Sucht- oder Rauschmitteltherapie, insbesondere der Therapie des Alkoholismus, **dadurch gekennzeichnet, daß** der Modulator oder zumindest einer der Modulatoren des cholinergen Systems ein Inhibitor der Acetylcholinesterase ist, der vorzugsweise aus der Gruppe ausgewählt ist, die Galanthamin und Desoxypeganin sowie deren pharmakologisch akzeptablen Salze und Derivate umfaßt.

2. Wirkstoff-Kombination nach Anspruch 1, **dadurch gekennzeichnet, daß** die antiexzitatorisch wirkende Substanz oder zumindest eine der antiexzitatorisch wirkenden Substanzen aus der Gruppe der NMDA-Rezeptorantagonisten ausgewählt ist, vorzugsweise aus der Gruppe ausgewählt ist, die Acamprosat und Memantine sowie deren pharmakologisch akzeptablen Salze und Derivate umfaßt.

3. Wirkstoff-Kombination nach Anspruch 1, **dadurch gekennzeichnet, daß** die antiexitatorisch wirkende Substanz ein Modulator metabotropar Glutamatrezeptoren ist, der vorzugsweise aus der Gruppe ausgewählt ist, die 3,5-Dihydro-3,5-dimethyl-6-(4-ethoxyphenyl)-2-(4-methanesulfonylamino-phenylsulfonyl)-2H-1,2-oxazin bzw. 2-(4-Acetylaminobenzen-sulfonyl)-3,6-dihydro-3,5-dimethyl-6-(4-methoxyphenyl)-2H-1,2-oxazin, 3-(3-Chlorobenzoylamino)-1-[2-(3-chlorophenyl)-ethyl]-3-methylpyrrolidin-2-thion und dessen pharmakologisch akzeptablen Derivate umfaßt.

4. Wirkstoff-Kombination nach einem der Ansprüche 1 bis 3, **dadurch gekennzeichnet, daß** sie als Arzneiform vorliegt, wobei die verabreichts Einzeldosis Galanthamin oder eines seiner pharmakologisch akzeptablen Salze oder Derivate vorzugsweise im Bereich von 1 - 50 mg liegt, oder die verabreichte Einzeldosis Desoxypeganin oder eines seiner pharmakologisch akzeptablen Salze oder Derivate vorzugsweise im Bereich von 10 - 500 mg liegt, und die verabreichte Einzeldosis Acamprosat oder eines seiner pharmakologisch akzeptablen Salze oder Derivate vorzugsweise im Bereich von 100 - 5000 mg liegt, oder die verabreichte Einzeldosis Memantine oder eines seiner pharmakologisch akzeptablen Salze oder Derivate vorzugsweise im Bereich von 1 - 50 mg liegt, oder die verabreichte Einzeldosis des Modulators metabotroper Glutamatrezeptoren vorzugsweise im Bereich von 0,1 - 100 mg liegt.

5. Wirkstoff-Kombination nach einem der vorhergehenden Ansprüche, **dadurch gekennzeichnet, daß** sie in Form einer Arzneiform vorliegt, die eine Depotwirkung aufweist.

6. Wirkstoff-Kombination nach einem der vorangehenden Ansprüche, **dadurch gekennzeichnet, daß** sie in Form eines oral zu verabreichenden Arzneimitteln vorliegt.

7. Wirkstoff-Kombination nach einem der vorangahenden Ansprüche, **dadurch gekennzeichnet, daß** sie in Form eines parenteral zu verabreichenden Arzneimittels vorliegt.

8. Wirkstoff-Kombination nach Anspruch 7, **dadurch gekennzeichnet, daß** sie in Form eines transdermal zu verabreichenden Arzneimittels vorliegt.

9. Varwendung einer Wirkstoff-Kombination nach einem der Ansprüche 1 bis 8 zur Herstellung einer Arzneiform zur medikamentösen Sucht- oder Rauschmitteltherapie, insbesondere der Therapie des Alkoholismus.

10. Verwendung einer Wirkstoff-Kombination nach einem der Ansprüche 1 bis 3 zur Herstellung einer Arzneiform zur medikamentösen Sucht- oder Rauschmitteltherapia, insbesondere der Therapie des Alkoholismus.

11. Verwendung nach Anspruch 10, **dadurch gekennzeichnet, daß** die Arzneiform in Form einer Darreichungsform hergestellt wird, die eine Depotwirkung aufweist.

12. Verwendung nach Anspruch 10 oder 11, **dadurch gekennzeichnet, daß** die Arzneiform in Form einer oralen Darreichungsform hergestellt wird.

13. Verwendung nach Anspruch 10 oder 11, **dadurch gekennzeichnet, daß** die Arzneiform in Form einer paranteralen Darreichungsform hergestellt wird.

14. Verwendung nach Anspruch 13, **dadurch gekennzeichnet, daß** die Arzneiform in Form einer transdermalen Darreichungsform hergestellt wird.

15. Verwendung nach einem der Ansprüche 10 bis 14, **dadurch gekennzeichnet, daß** die Arzneiform eine zu verabreichende Einzaldosis von Galanthamin oder eines seiner pharmakologisch akzeptablen Salze odar Derivate vorzugsweise im Bareich von 1 - 50 mg aufweist, oder eine zu verabreichende Einzeldosis von Desoxypeganin oder eines seiner pharmakologisch akzeptablen Salze oder Derivate vorzugsweise im Bereich von 10 - 500 mg aufweist, und eine zu verabreichende Einzeldosis von Acamprosat oder eines seiner pharmakologisch akzeptablen Salze oder Derivate vorzugsweise im Bereich von 100 - 5000 mg aufweist, oder eine zu verabreichande Einzeldosis von Memantine oder eines seiner pharmakologisch akzeptablen Salze oder Derivate vorzugsweise im Bereich von 1 - 50 mg aufweist, oder eine zu verabreichande Einzeldosis des Modulators metabotroper Glutamatrazeptoren vorzugsweise im Bereich von 0,1 - 100 mg aufweist.

## Claims

1. Active ingredient combination composed of at least one modulator of the cholinergic system with at least one substance having antiexcitatory activity for pharmacological addictive substance or intoxicant therapy, in particular the therapy of alcoholism, **characterized in that** the modulator or at least one of the modulators of the cholinergic system is an inhibitor of acetylcholinesterase which is preferably selected from the group comprising galanthamine and deoxypeganine and the pharmacologically acceptable salts and derivatives thereof.

2. Active ingredient combination according to Claim 1, **characterized in that** the substance having antiexcitatory activity or at least one of the substances having antiexcitatory activity is selected from the group of NMDA receptor antagonists, preferably selected from the group which comprises acamprosate and memantine and the pharmacologically acceptable salts and derivatives thereof.

3. Active ingredient combination according to Claim 1, **characterized in that** the substance having antiexcitatory activity is a modulator of metabotropic glutamate receptors which is preferably selected from the group which comprises 3,6-dihydro-3,5-dimethyl-6-(4-ethoxyphenyl)-2-(4-methanesulphonylaminophenylsulphonyl)-2H-1,2-oxazine and 2-(4-acetylaminobenzenesulphonyl)-3,6-dihydro-3,5-dimethyl-6-(4-methoxyphenyl)-2H-1,2-oxazine; 3-(3-chlorobenzoylamino)-1-[2-(3-chlorophenyl)-ethyl]-3-methylpyrrolidine-2-thione and its pharmacologically acceptable derivatives.

4. Active ingredient combination according to any of Claims 1 to 3, **characterized in that** it is in the form of a pharmaceutical form where the administered single dose of galanthamine or one of its pharmacologically acceptable salts or derivatives is preferably in the range 1-50 mg, or the administered single dose of deoxypeganine or one of its pharmacologically acceptable salts or derivatives is preferably in the range 10-500 mg, and the administered single dose of acamprosate or one of its pharmacologically acceptable salts or derivatives is preferably in the range 100-5000 mg, or the administered single dose of memantine or one of its pharmacologically acceptable salts or derivatives is preferably in the range 1-50 mg, or the administered single dose of the modulator of metabotropic glutamate receptors is preferably in the range 0.1-100 mg.

5. Active ingredient combination according to any of the preceding claims, **characterized in that** it is in the form of a pharmaceutical form which has a depot effect.

6. Active ingredient combination according to any of the preceding claims, **characterized in that** it is in the form of a medicament to be administered orally.

7. Active ingredient combination according to any of the preceding claims, **characterized in that** it is in the form of a medicament to be administered parenterally.

8. Active ingredient combination according to Claim 7, **characterized in that** it is in the form of a medicament to be administered transdermally.

9. Use of an active ingredient combination according to any of Claims 1 to 8 which is intended for pharmacological addictive substance or intoxicant therapy, in particular the therapy of alcoholism, for producing a pharmaceutical form.

10. Use of an active ingredient combination according to any of Claims 1 to 3 for producing a pharmaceutical form for pharmacological addictive substance or intoxicant therapy, in particular the therapy of alcoholism.

11. Use according to Claim 10, **characterized in that** the pharmaceutical form is produced in the form of a dosage form which has a depot effect.

12. Use according to Claim 10 or 11, **characterized in that** the pharmaceutical form is produced in the form of an oral dosage form.

13. Use according to Claim 10 or 11, **characterized in that** the pharmaceutical form is produced in the form of a parenteral dosage form.

14. Use according to Claim 13, **characterized in that** the pharmaceutical form is produced in the form of a transdermal dosage form.

15. Use according to any of Claims 11 to 14, **characterized in that** the pharmaceutical form comprises a single dose for administration of galanthamine or one of its pharmacologically acceptable salts or derivatives is preferably in the range 1-50 mg, or a single dose for administration of deoxypeganine or one of its pharmacologically acceptable salts or derivatives is preferably in the range 10-500 mg, and a single dose for administration of acamprosate or one of its pharmacologically acceptable salts or derivatives is preferably in the range 100-5000 mg, or a single dose for administration of memantine or one of its pharmacologically acceptable salts or derivatives is preferably in the range 1-50 mg, or a single dose for administration of the modulator of metabotropic glutamate receptors is preferably in the range 0.1-100 mg.

## Revendications

1. Combinaison de substances actives comprenant au moins un modulateur du système cholinergique avec au moins une substance à effet anti-excitant pour la thérapie médicamenteuse de la toxicomanie ou de la dépendance aux stupéfiants, en particulier la thérapie de l'alcoolisme, **caractérisée en ce que** le modulateur ou au moins un des modulateurs du système cholinergique est un inhibiteur de l'acétylcholinestérase qui est de préférence choisi dans le groupe qui comprend la galanthamine et la désoxypéganine ainsi que leurs sels et dérivés pharmacologiquement acceptables.

2. Combinaison de substances actives selon la revendication 1, **caractérisée en ce que** la substance à effet anti-excitant ou au moins une des substances à effet anti-excitant est choisie dans le groupe des antagonistes du récepteur de NMDA, de préférence dans le groupe comprenant l'acamprosate et la mémantine ainsi que leurs sels et dérivés pharmacologiquement acceptables.

3. Combinaison de substances actives selon la revendication 1, **caractérisée en ce que** la substance à effet anti-excitant est un modulateur des récepteurs métabotropes du glutamate, qui est de préférence choisi dans le groupe comprenant la 3,6-dihydro-3,5-diméthyl-6-(4-éthoxyphényl)-2-(4-méthanesulfonylaminophénylsulfonyl)-2H-1,2-oxazine ou la 2-(4-acétylaminobenzènesulfonyl)-3,6-dihydro-3,5-diméthyl-6-(4-méthoxyphényl)-2H-1,2-oxazine; la 3-(3-chlorobenzoylamino)-1-[2-(3-chlorophényl)éthyl]-3-méthylpyrrolidine-2-thione et leurs dérivés pharmacologiquement acceptables.

4. Combinaison de substances actives selon l'une quelconque des revendications 1 à 3, **caractérisée en ce qu'**elle se trouve sous forme de médicament, la dose individuelle administrée de galanthamine ou d'un de ses sels ou dérivés pharmacologiquement acceptables se situant de préférence dans la plage de 1 à 50 mg, ou la dose individuelle administrée de désoxypéganine ou d'un de ses sels ou dérivés pharmacologiquement acceptables se situant de préférence dans la plage de 10 à 500 mg et la dose individuelle administrée d'acamprosate ou d'un de ses sels ou dérivés pharmacologiquement acceptables se situant de préférence dans la plage de 100 à 5000 mg ou la dose individuelle administrée de mémantine ou d'un de ses sels ou dérivés pharmacologiquement acceptables se situant de préférence dans la plage de 1 à 50 mg ou la dose individuelle administrée du modulateur des récepteurs métabotropes du glutamate se situant de préférence dans la plage de 0,1 à 100 mg.

5. Combinaison de substances actives selon l'une quelconque des revendications précédentes, **caractérisée en ce qu'**elle se trouve sous forme d'un médicament qui présente un effet retard.

6. Combinaison de substances actives selon l'une quelconque des revendications précédentes, **caractérisée en ce qu'**elle se trouve sous forme d'un médicament à administrer par voie orale.

7. Combinaison de substances actives selon l'une quelconque des revendications précédentes, **caractérisée en ce qu'**elle se trouve sous forme d'un médicament à administrer par voie parentérale.

8. Combinaison de substances actives selon la revendication 7, **caractérisée en ce qu'**elle se trouve sous forme d'un médicament à administrer par voie transdermique.

9. Utilisation d'une combinaison de substances actives selon l'une quelconque des revendications 1 à 8 pour la préparation d'une forme de médicament pour la thérapie médicamenteuse de la toxicomanie ou de la dépendance aux stupéfiants, en particulier la thérapie de l'alcoolisme.

10. Utilisation d'une combinaison de substances actives selon l'une quelconque des revendications 1 à 3 pour la préparation d'une forme de médicament pour la thérapie médicamenteuse de la toxicomanie ou de la dépendance aux stupéfiants, en particulier la thérapie de l'alcoolisme.

11. Utilisation selon la revendication 10, **caractérisée en ce que** la forme de médicament est préparée sous forme d'une forme d'administration qui présente un effet retard.

12. Utilisation selon la revendication 10 ou 11, **caractérisée en ce que** la forme de médicament est préparée sous forme d'une forme d'administration par voie orale.

13. Utilisation selon la revendication 10 ou 11, **caractérisée en ce que** la forme de médicament est préparée sous forme d'une forme d'administration par voie parentérale.

14. Utilisation selon la revendication 13, **caractérisée en ce que** la forme de médicament est préparée sous forme d'une forme d'administration par voie transdermique.

15. Utilisation selon l'une quelconque des revendications 10 à 14, **caractérisée en ce que** la forme de médicament présente une dose individuelle à administrer de galanthamine ou d'un de ses sels ou dérivés pharmacologiquement acceptables de préférence dans la plage de 1 à 50 mg, ou une dose individuelle à administrer de désoxypéganine ou d'un de ses sels ou dérivés pharmacologiquement acceptables de préférence dans la plage de 10 à 500 mg et une dose individuelle à administrer d'acamprosate ou d'un de ses sels ou dérivés pharmacologiquement acceptables de préférence dans la plage de 100 à 5000 mg ou une dose individuelle à administrer de mémantine ou d'un de ses sels ou dérivés pharmacologiquement acceptables de préférence dans la plage de 1 à 50 mg ou une dose individuelle à administrer du modulateur des récepteurs métabotropes du glutamate de préférence dans la plage de 0,1 à 100 mg.
